# EUROPEAN PATENT SPECIFICATION

(11) **EP 1 479 361 B1**
(45) Date of publication and mention of the grant of the patent: **08.09.2010**
(21) Application number: 03011325.2
(22) Date of filing: 19.05.2003
(51) Int. Cl.: A61F 13/15, A61L 15/46, A61L 15/20

(54) **Volatile material-containing sanitary absorbent article with barrier package**
Flüchtiges Material enthaltender absobierender Hygieneartikel mit Sperrschichtverpackung
Article absorbant hygiènique contenant un matériau volatile avec un emballage imperméable

(43) Date of publication of application: 24.11.2004
(73) Proprietor: THE PROCTER & GAMBLE COMPANY, Cincinnati, OH 45202 (US)
(72) Inventor: Guerreschi, Lisa, 26048 Sospiro, Cremona (IT); Sierri, Giancarlo, 65105 Montesilvano (IT); Massacesi, Ettore, 65016 Montesilvano - Pescara (IT); Partenza, Vincenzo, 65010 Elice - Pescara (IT); Carlucci, Giovanni, 66100 Chieti (IT)
(74) Representative: Briatore, Andrea

(56) References cited:
- EP-A- 0 931 646
- EP-A- 0 963 748
- EP-A- 1 250 940
- EP-A- 1 250 941
- EP-A- 1 300 238
- WO-A-98/32601
- WO-A-03/006237
- US-A- 3 963 029
- US-A- 4 556 146
- US-A- 4 765 477
- US-A- 5 116 649
- US-A- 5 266 592
- US-A- 5 451 404
- US-A1- 2002 150 704

## Description

### FIELD OF THE INVENTION

The present invention relates to the field of sanitary absorbent articles for feminine hygiene of human wearers. In particular, the present invention relates to sanitary absorbent articles comprising non-aqueous volatile material, which has the tendency to vaporize at room temperature. These articles are packaged according to the present invention in a package, which is substantially impervious to liquids and vapours. This ensures that the amount of the non-aqueous volatile material initially present in the absorbent article does not substantially decrease during a longer period of storage or shelf life of the packaged article.

### BACKGROUND OF THE INVENTION

Recent consumer research activities have uncovered the need for certain absorbent articles of personal hygiene containing functional non-aqueous materials having a tendency to vaporize and evaporate at room temperature. Examples of such articles are sanitary napkins containing quantities of substances delivering a cooling impression on human skin, such as menthyl lactate as disclosed in European patent applications EP 1,250,940 A1 and EP 1,250,941 A1. However, since such non-aqueous volatile materials like menthyl lactate have the tendency to vaporize at room temperature a need exists for reducing or even preventing their evaporation from the article. Otherwise the amount of menthyl lactate would quickly decrease so that the article would not offer the intended functionality anymore after already a short period of storage or shelf life.

In the prior art there are numerous references known with respect to packaging sanitary absorbent articles, such as sanitary napkins or the like. Examples are for instance GB 2,167,382, US 4,556,146 or US 5,423,568. However, all these references known so far are directed to packaging absorbent articles, which do not contain volatile material and thus also no packages for reducing or even preventing the evaporation of such volatile materials are suggested. All that is known so far in this field are numerous ways and configurations for packaging articles but only conventional materials like polyethylene (PE) are suggested therefore, if any.

US 3,963,029 discloses a diaper containing a volatile material. The diaper is folded in a way such that only the backsheet of the diaper is exposed to the outside. The backsheet is then sealed, thereby preventing escape of vapour from the interior of the diaper. There is no disclosure in this reference of a package for containing the diaper.

Vis-a-vis the above-cited prior art it is the object of the present invention to provide an absorbent article for personal, in particular feminine hygiene containing a non-aqueous volatile material, which is packaged in a package reducing or even preventing evaporation of said non-aqueous volatile material. This is needed to preserve the article's functionality provided by the non-aqueous volatile material. More particularly, it is an object of the present invention to provide an absorbent article for personal, in particular feminine hygiene containing non-aqueous volatile material, wherein the intended functionality is maintained for a prolonged period of storage and or shelf life.

It is another object of the present invention to provide an absorbent article for feminine hygiene containing a non-aqueous volatile material, which is packaged in a package that can be opened by a user very conveniently.

It is a further object of the present invention to provide an absorbent article for feminine hygiene containing a non-aqueous volatile material, which is packaged in a package that can be used as a disposal pouch for a used article.

A still further object of the present invention is to provide an absorbent article for feminine hygiene containing a non-aqueous volatile material, which is packaged in a package comprising only one interconnected piece of package material.

### SUMMARY OF THE INVENTION

The present invention provides a packaged absorbent article for feminine hygiene selected from a sanitary napkin, a panty liner, an interlabial device, a tampon or an incontinence device. The absorbent article is packaged in a vapour-impermeable package made of a package material completely enclosing the article and being able to prevent contact of said absorbent article with the outside environment. The absorbent article comprises a non-aqueous volatile material having a vapour pressure at 20°C of at least 0.01 mbar, and the package material has a thickness comprised between 0.020 and 1 millimeters and a basis weight comprised between 8.0 and 60.0 g/m² and further comprises at least one barrier layer made of a material selected from the group consisting of ethyl vinyl alcohol resins, aluminised film and aluminum foil, oriented polyethylene terephtalate, glycol-modified polyethylene terephtalate, oriented polyamide, aromatic polyamide, polymeric films being coated with volatile-impermeable lacquers or mixtures thereof. The package material has a permeability with respect to menthyl lactate of not more than 25 % according to the permeability test described herein.

In a particular preferred embodiment the package comprises four layers of LDPE (low density polyethylene) and one layer of EVOH (ethyl vinyl alcohol) in the middle of the other four layers, thus 2 LDPE layers being arranged above the EVOH layer and the two other LDPE layers being arranged below the EVOH layer.

### BRIEF DESCRIPTION OF THE DRAWINGS

Figure 1 illustrates the closure of the free end edge of a preferred embodiment of the package of the present invention by an adhesive.
Figures 2a-c illustrate the procedure of closing the package around an exemplary sanitary napkin according to a preferred embodiment of the present invention.
Figures 3-6 illustrate the test method described herein for determining the vapour permeability of package materials.

### DETAILED DESCRIPTION OF THE INVENTION

The term 'absorbent article' is used herein in a very broad sense including any article able to receive and/or absorb and/or contain and/or retain bodily fluids and/or bodily exudates. Absorbent articles as referred to herein are disposable. Typically, absorbent articles according to the present invention have a liquid-pervious topsheet as the in-use wearer-facing surface, a liquid-impervious backsheet as the in-use garment-facing surface and an absorbent core interposed between said topsheet and said backsheet. Optionally, absorbent articles according to the present invention can also comprise a so-called secondary topsheet, which is located between said topsheet and said absorbent core. The absorbent articles, which are referred to in the present invention are preferably sanitary napkins, panty liners or incontinence devices. Other absorbent articles like tampons or interlabial devices are also encompassed herein. Particularly preferred absorbent articles in the context of the present invention are disposable absorbent articles.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is actually put in contact with the anatomy of the user.

The absorbent article of the present invention comprises a non-aqueous volatile material as a functional ingredient. Said volatile material can be present at any location of the article. In preferred embodiments said non-aqueous volatile material is present on the topsheet and/or on the secondary topsheet. In another preferred embodiment said non-aqueous volatile material is present in said absorbent core. The term 'non-aqueous volatile material' as used herein refers to a substantially water-free material, which has a vapour pressure at 20°C of at least 0.01 mbar.

Examples for such materials are cooling agents, like menthyl lactate, which is a solid at room temperature and has a vapour pressure of about 0.05 mbar at 20°C. Said non-aqueous volatile material provides the absorbent article with the functionality to convey a perception to the wearer of the article. In the case of menthyl lactate the absorbent article is provided with the capability of delivering a cooling impression to the wearer as outlined in EP 1,250,940 and A1, EP 1,250,941 A1.

Other non-aqueous volatile materials for use herein include, but are not limited to perfumes and fragrances , provide the absorbent article with a pleasant smell, which is perceivable by the wearer.

Examples of non-aqueous volatile material suitable for use herein, namely cooling agents, are compounds from the chemical classes of ketals, carboxamides, cyclohexanol derivatives and cyclohexyl derivatives. Other examples are camphor, borneol, eucalyptol, methyl salicylate, tea tree oil, eucalyptus oil, peppermint oil, menthyl lactate and mixtures thereof. Examples for each of the aforementioned chemical classes suitable for use as the non-aqueous volatile material are described in more detail hereinafter. The amount of the non-aqueous volatile material present in an absorbent article of course depends from various factors, such as type of article, type of non-aqueous volatile material, intended functionality of the non-aqueous volatile material etc. Absorbent articles of the present invention typically comprise from 0.1 mg to 100 mg, preferably from 1 mg to 80 mg of non-aqueous volatile material. As an example a sanitary napkin comprising a cooling agent, preferably menthyl lactate for the purpose of providing the wearer with a cooling impression on the skin contains cooling agent in an amount of from 3 mg to 50 mg, typically from 15 mg to 40 mg.

Suitable ketals for use as the non-aqueous volatile material according to the present invention have the following formula: 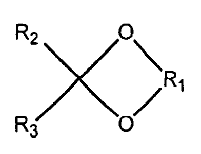 wherein which R¹ represents a C₂-C₆-alkylene radical having at least 1, but not more than 3 hydroxyl group(s), preferably 1 hydroxyl group, and either R² and R³ independently of one another represent C₁-C₁₀-alkyl which is optionally substituted by 1 to 3 radicals selected from the group comprising hydroxyl, amino and halogen (such as fluorine, chlorine, bromine or iodine), C₅-C₇-cycloalkyl, preferably cyclohexyl, C₆-C₁₂-aryl, preferably phenyl, with the proviso that the total number of the C atoms of R² and R³ is not less than 3, or R² and R³ together represent an alkylene radical which, together with the carbon atom which carries the radicals R² and R³, forms a 5-7-membered ring, while it is possible for this alkylene radical, in turn, to be substituted by C₁-C₆-alkyl groups, or mixtures thereof.

Suitable carboxamides for use as the non-aqueous volatile material according to the present invention have the following formula: 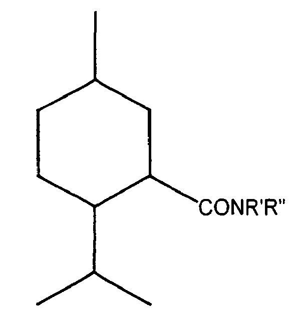 wherein R', when taken separately, is hydrogen or an aliphatic radical containing up to 25 carbon atoms; R" when taken separately is hydroxy, or an aliphatic radical containing up to 25 carbon atoms, with the proviso that when R' is hydrogen R" may also be an aryl radical of up to 10 carbon atoms and selected from the group consisting of substituted phenyl, phenalkyl or substituted phenalkyl, naphthyl and substituted naphthyl, pyridyl; and R' and R", when taken together with the nitrogen atom to which they are attached, represent a cyclic or heterocyclic group of up to 25 carbon atoms, or mixtures thereof.

Another class of carboxiamides for use as the non-aqueous volatile material according to the present invention have the following formula: 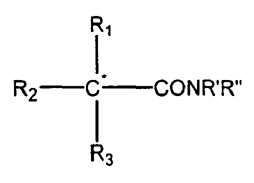 wherein R' and R", when taken separately, are each hydrogen, C₁-C₅ alkyl or C₁-C₈ hydroxyalkyl and provide a total of no more than 8 carbon atoms, with the proviso that when R' is hydrogen R" may also be alkylcarboxyalkyl of up to 6 carbon atoms; R' and R", when taken together, represent an alkylene group of up to 6 carbon atoms, the opposite ends of which group are attached to the amide nitrogen atom thereby to form a nitrogen heterocycle, the carbon chain of which may optionally be interrupted by oxygen; R₁ is hydrogen or C₁-C₅ alkyl; and R₂ and R₃ are each C₁-C₅ alkyl; with the provisos that (i) R₁, R₂ and R₃ together provide a total of at least 5 carbon atoms, preferably from 5-10 carbon atoms; and (ii) when R₁ is hydrogen, R₂ is C₂-C₅ alkyl and R₃ is C₃-C₅ alkyl and at least one of R₂ and R₃ is branched, preferably in an alpha or beta position relative to the carbon atom marked (*) in the formula, or a mixture thereof.

Examples for cyclohexanol derivatives for use as the non-aqueous volatile material according to the present invention have the following formula: 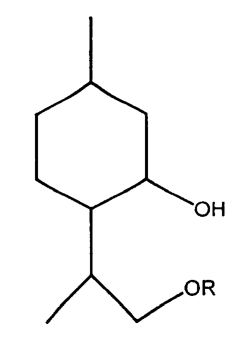 wherein R represents a linear or branched alkyl group having 1 to 5 carbon atoms.

Another class of cyclohexanol derivatives for use as the non-aqueous volatile material according to the present invention have the following formula: 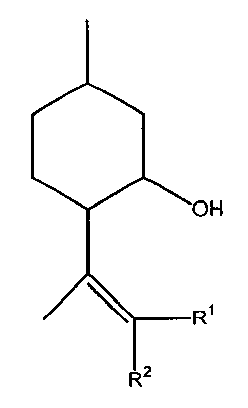 wherein R¹ and R² are independently hydrogen, or a linear or branched alkyl group having 1 to 5 carbon atoms, or mixtures thereof.

Examples for cyclohexyl derivatives for use as the non-aqueous volatile material according to the present invention have the following formula: 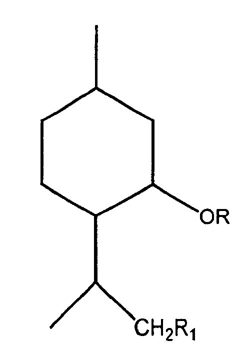 wherein R represents hydrogen, a C₁-C₅ linear or branched alkyl group, a C₁-C₅ alkenyl group, a C₁-C₅ alkoxy group or a C₁-C₅ acyloxy group, R₁ represents hydrogen, or a linear or branched alkyl group having from 1 to 5 carbon atoms.

It is also within the scope of the present invention to use mixtures of the aforementioned compound classes.

In a preferred embodiment said non-aqueous volatile material is a cooling agent, which comprises a combination of (a) menthol and/or peppermint oil and (b) a second cooling agent selected from the group consisting of ketals, carboxamides, cyclohexyl derivatives with the exception of menthol, cyclohexanol derivatives and mixtures thereof, preferably the second cooling agent is a cyclohexyl derivative according to following formula: 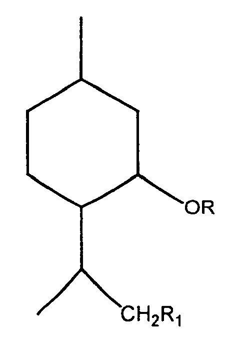 wherein R represents hydrogen, a C₁-C₅ linear or branched alkyl group, a C₁-C₅ alkenyl group, a C₁-C₅ alkoxy group or a C₁-C₅ acyloxy group, R₁ represents hydrogen, or a linear or branched alkyl group having from 1 to 5 carbon atoms, with the exception of R and R₁ both being hydrogen, or mixtures thereof, and most preferably is menthyl lactate, typically in a weight ratio of (a) to (b) from 1/1 to 1/100. Suitable examples for the compound classes mentioned in this paragraph can be found *supra.*

The article may comprise a delivery system for releasably containing and delivering the volatile material to at least a portion of the skin and/or mucosal surface of the wearer of the article. The delivery system may be of any configuration including, but not limited to, one that contains the volatile material in powder, particle or flake form, or in a solution, a dispersion, a suspension, an emulsion or the like. The delivery system may comprise a structure such as a microcapsule, an absorbent material, a nano-phase particulate structure, a cell, an adhesive, a solid support, or the like or a composition such as an emollient-containing composition. Preferably the delivery system is an emollient-containing composition. In a particularly preferred embodiment the cooling agent like menthyl lactate is applied to the article using polyethylene glycol as a carrier, particularly PEG-400. Typically, the weight ratio of menthyl lactate to PEG-400 is 30:70 in such mixtures. Preferably the delivery system positions the active in proximity to the skin during wear of the article and, more preferably, onto at least a portion of the skin and/or mucosal surface of the wearer of the article.

According to the present invention the absorbent article described herein is packaged in a package, which reduces or even prevents evaporation of the non-aqueous volatile material out of the absorbent article, such that said article is capable to retain the intended functionality also over a prolonged shelf life. The package is made of a package material, which can be a single-layered material and is preferably a multi-layered material. Even more preferably the package material is a co-extruded multi-layered material, which is produced by any suitable process known in the art.

The package herein has an 'inner surface', being directed away from the outside environment, i.e. the inner surface is the surface of the package directly adjacent to the article it contains. In the embodiment herein where the package is made from a multi-layered package material said inner surface of said package is provided by the innermost surface of the innermost layer of said package material, which is closest to the packaged article. In the embodiment herein, where the package material is provided by only one layer, said inner surface is provided by the inner surface of said package material, being directed towards the packaged article.

The package herein has an 'outer surface', being oriented towards the outside environment. In the embodiment herein where the package is made from a multi-layered package material said outer surface of said package is provided by the outermost surface of the outermost layer of said package material, which is furthest away from the packaged article. Otherwise, in case the package material is provided by only one layer, said outer surface is provided by the outer surface of said package material, being directed away from the packaged article and is directly adjacent to the outside environment.

As an essential feature the package material of the present invention has a permeability of not more than 25 %, preferably not more than 15% and more preferably not more than 5% according to the test method described herein with respect to menthyl lactate. The package material preferably comprises at least one 'barrier layer'. A barrier layer as used herein reduces or even prevents evaporation of said non-aqueous volatile material out of said package. Suitable materials for use as barrier layer herein include but are not limited to layers made of ethylene vinyl alcohol resins (EVOH); metallised, particularly aluminised plastic films; metal foils; oriented polyethylene terephtalate (PET); PETG (glycol-modified PET); oriented polyamide; aromatic polyamide; or polymeric films like polyethylene films with special lacquer coatings, which provide the polymeric film with the vapour barrier functionality. There are a number of well-known techniques for forming such package materials including, but not limited to casting and blowing technology. The package material can be made of a monolayer or a multilayered structure of e.g. 5 coextensive layers in a preferred embodiment. Typically, the package material will have a thickness between about 0.020 and about 1.0 millimetres, a basis weight of between about 18.0 and 60.0 g/m². The package material can have an embossing texture or a specific pattern design. The package material could be, without limitation, printed on one side and/ or pigmented, with release function on one side by using e.g. silicone, but not limited thereto, coated, spray and by using other suitable technology. The silicon can be applied on the package material surface with specific dimensioned area coverage and/or with full area coverage. The release properties could be also achieved by adding the release agent already incorporated in the resins and directly extruded during the package material formation. The package material could be also without release properties. In a preferred multi-layered package material of the present invention the barrier layer is preferably located between the layers providing the inner and outer surfaces of the package material. In other embodiments, the barrier layer provides the inner surface of the package material, being oriented towards the packaged article. Package materials conventionally used for packaging of absorbent articles, such as polyethylene (PE), polypropylene (PP) or polyvinyl chloride (PVC) are not suitable as barrier layer herein, since they allow e.g. menthyl lactate to evaporate out of the package nearly completely within a time frame of about 2 weeks.

As outlined *supra,* apart from the barrier layer the package material for use with the present invention preferably comprises further layers. These further layers can be either additional barrier layers or layers made of materials, which are conventionally used as packaging material for absorbent articles, e.g. PE or PP. One particularly preferred package material comprises four layers of LDPE and one layer of EVOH in the middle of the other four layers, thus 2 LDPE layers being arranged above the EVOH layer and the two other LDPE layers being arranged below the EVOH layer. Such a package material is commercialised by Nordenia under the code 6261BK without silicone coating or 6261SE with silicone coating on one surface. The individual layers of the Nordenia 6261 package material have a thickness of about 5 µm each. The Nordenia 6261 materials have the benefit of very high impermeability towards non-aqueous volatile material thanks to the barrier performance of the EVOH layer on one hand and good sealability and/or crimpability thanks to the PE layers arranged on the outer surfaces of the packaging material on the other.

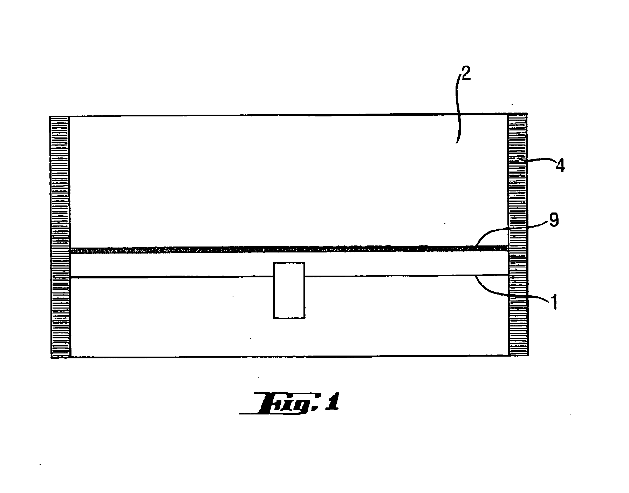

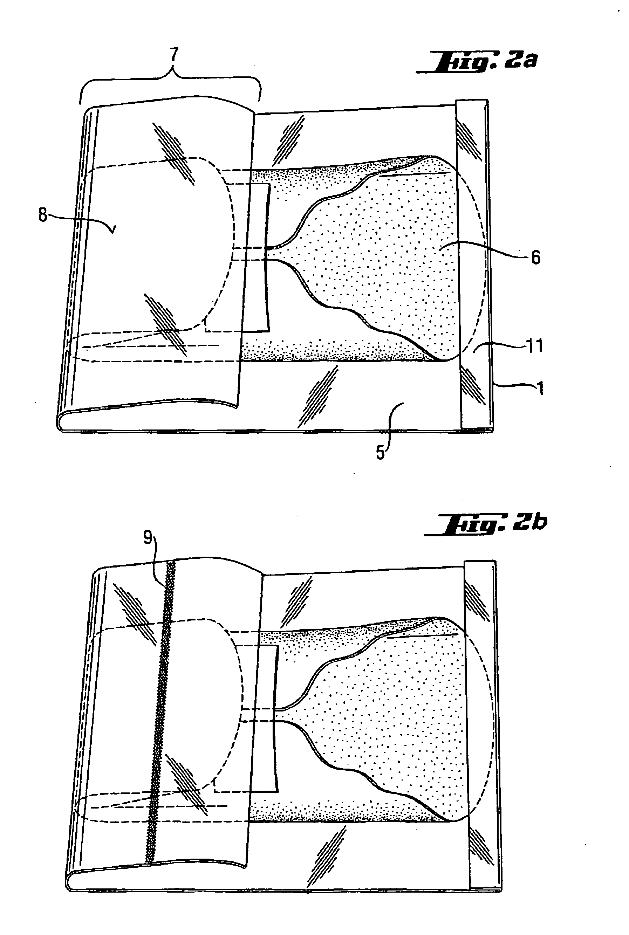

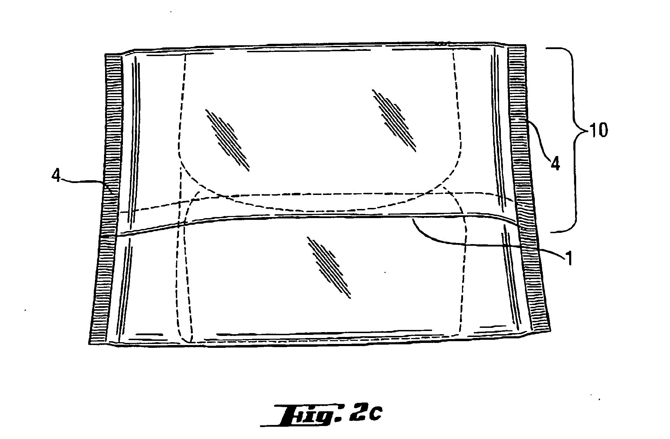

For illustrating a particularly preferred manner of arranging the package material around the absorbent article in order to form the package of an exemplary packaged sanitary napkin is described. The procedure is similar to those disclosed in e.g. US 4,556,146 or US 5,413,568. The sanitary napkin is therefore provided onto a substantially rectangular sheet of package material having longitudinal edges on its longer peripheral sides and end edges on its shorter peripheral sides. The sanitary napkin is placed onto the sheet of package material so that the longitudinal edges of the napkin, i.e. the longer side edge, are placed parallel to the longitudinal edges of the piece of package material. The package material has a size large enough so that it extends beyond the perimeter of the sanitary napkin on all sides of the napkin. The longitudinal edge portions of the package material extending beyond the longitudinal perimeter sides of the sanitary napkin are called hereinafter flap portions. The sanitary napkin and the package material together are then folded lengthwise into thirds about two fold axes, which are extending laterally across the sanitary napkin. Subsequently to this step the flap portions of the package material are sealed together in order to close the package along the longitudinal edges of the article. The sealing can be facilitated by heat-sealing, by crimp sealing or by using a suitable adhesive, which does not detriment the liquid- and vapour-imperviousness of the package, e.g. Findley H2674 or Fuller HL 1719. Crimp sealing is widely known, it means bonding two materials by a combination of heat and mechanical entangling. After this sealing step the package is open only on one side, which is the exposed free end edge of the package material. This free end edge is according to the present invention preferably sealed to the surface of the portion of the package material it overlies by an adhesive. Of course the adhesive used for this purpose must not detriment the fluid and vapour-impermeability properties of the package. As already outlined before, Findley H2674 and Fuller HL 1719 have the characteristics required herein. Another procedure to seal the free end edge of the package material is heat-sealing to the underlying portion of the package material. This requires that both inner and outer surfaces of the package material are heat-sealable. By completing this step of closing the package on the free end edge of the package material the package is completely closed around the sanitary napkin. Figure 1 illustrates an example for the above-described embodiment, where the free end edge (1) of the package (2) is sealed by a line of adhesive (9). The flap portions (4) of the package material are sealed by crimp sealing. Alternatively, the sealing of the free end edge (1) can be achieved by a crimp sealing line instead of the line of adhesive. Figures 2a-c illustrate the procedure of arranging the package material (5) around an exemplary sanitary napkin (6) as described above. In Figure 2a about one third (7) of the package material (5) together with the sanitary napkin (6) is folded over the first folding axis. In Figure 2b on the outer surface (8) of the package material (5) in the one third (7), which was folded over the first folding axis is provided with a line of adhesive (9). In Figure 2c another third (10) of the package material (5) is folded together with the sanitary napkin (6) across the second folding axis over the outer surface (8) of the third (7) of package material (5), which was folded across the first folding axis before. The free end edge (1) of the package material (5) is secured to the outer surface (8) of the package material (5) by said line of adhesive (9). In a particularly preferred execution the free end edge (1) is folded over itself at about 0.5 cm length in the way as illustrated in Figures 2a, reference numeral (11). This allows securing the folded area (11) of the free end edge (1) to the line of adhesive (9). Finally, the package is sealed along the longitudinal edges by an adhesive or by crimp sealing, thereby forming the flap portions (4).

In another alternative embodiment a package material is used, which is siliconized except the flap portions. The flap portions can then be sealed by a suitable adhesive as described herein.

The above-described embodiment of the package according to the present invention can optionally comprise opening aids, such as grasping means in the form of flaps or fins or the like, which increase ease of opening of the package by improving accessibility of the exposed end edge of the package material. Generally, the above embodiment is opened by pulling on the free end edge (1) of the package material (5). In this course, the sealing by the line of adhesive breaks (9) and the package material (5) and thus also the packaged article (6) can be unfolded wherewhile the sealing along the flap portions (4) of the package material (5) breaks, too.

Another advantage of the above-described embodiment is that the package is constituted by only one interconnected piece of package material. Thus, the number of waste parts after having opened the package is minimized. Furthermore, the package described above can also be used as a wrapper for the hygienic disposal of a used absorbent article.

Another option of forming the package by enclosing the absorbent article in package material is a "pouch"-type package similar to the one as disclosed in e.g. WO 02/08087. The packaging material is a U-shaped member formed of a rectangular or square piece of suitable package material. The U-shaped member comprises a first marginal edge and a second marginal edge, which overly each other and abut each other, a third marginal edge and a forth marginal edge, which overly each other and abut each other, and a fifth marginal edge and sixth marginal edge, which overly each other and abut each other. The bottom panel is located parallel to and is spaced from the top panel by an end panel. All of the panels are made up of respective portions of the piece of package material forming the U-shaped member. The absorbent article is located within the interior of the package, i.e., between the top and the bottom panels. The top and the bottom panels are sealed together along their abutting marginal edges, i.e. first and second marginal edge, third and forth marginal edge, and fifth and sixth marginal edge to hold the absorbent article within the package. The sealing can be facilitated by heat-sealing, by crimp sealing, by using a suitable adhesive, which does not detriment the liquid- and vapour-imperviousness of the package (e.g. Findley H2674, Fuller HL 1719).

Another alternative "pouch" package option can be described by two members of package material of the same size and shape, wherein the absorbent article is placed on one of said members and the other of said members is placed on the absorbent article in a manner coextensive and congruent with the first member of package material. Both members of package material are then sealed to each other along their peripheral edge, whereby enclosing the absorbent article. In this configuration the packaging material is sealed on all four sides. The sealing can be facilitated by heat-sealing, by crimp sealing, by using a suitable adhesive, which does not detriment the liquid- and vapour-imperviousness of the package (e.g. Findley H2674, Fuller HL 1719).

The above "pouch" embodiments of the package according to the present invention optionally comprise opening aids, such as tearing aids like notches, weakness lines, rows of weakness points and the like, all in the abutting edges, or tear tapes increasing ease of opening of the package. Of course those opening aids must not detrimentally affect the vapour barrier functionality.

Furthermore, in all specific embodiments described herein before it may be desirable to round the edges of the package in the sealed areas for making the packaged article more convenient. Since the package is relatively hard in the sealed areas unrounded edges could be painful for the user upon handling or carrying.

According to the present invention it is possible to package one individual absorbent article in a package as described herein or a higher number of absorbent articles in one common package. It is for instance useful to pack a certain number of sanitary napkins according to the present invention, which fits the number of sanitary napkins used during one day in the menstruation period of a woman, into one common package.

### Test methods

### Headspace test for determining the permeability of package materials for menthyl lactate (ML)

### 1.Apparatus

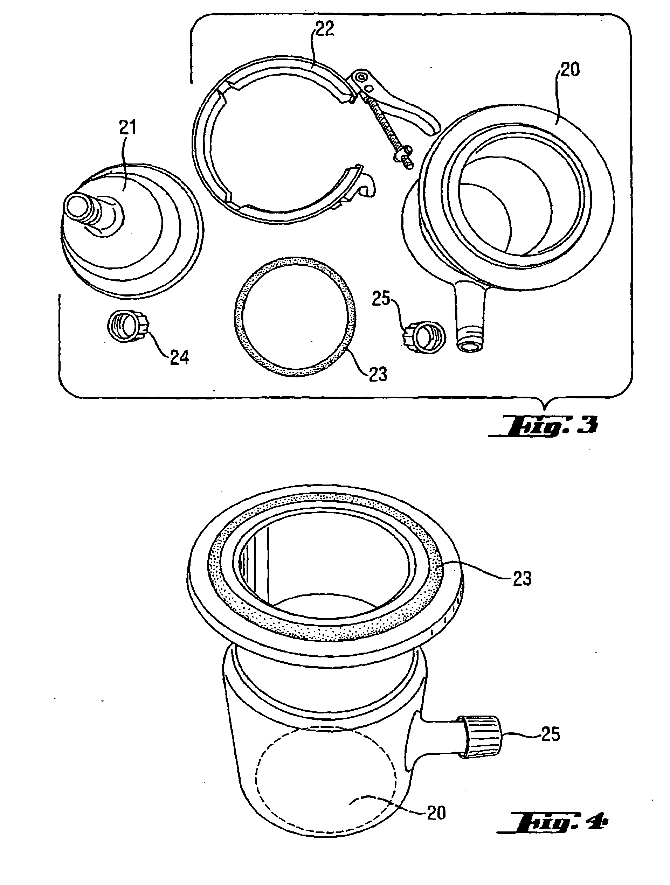

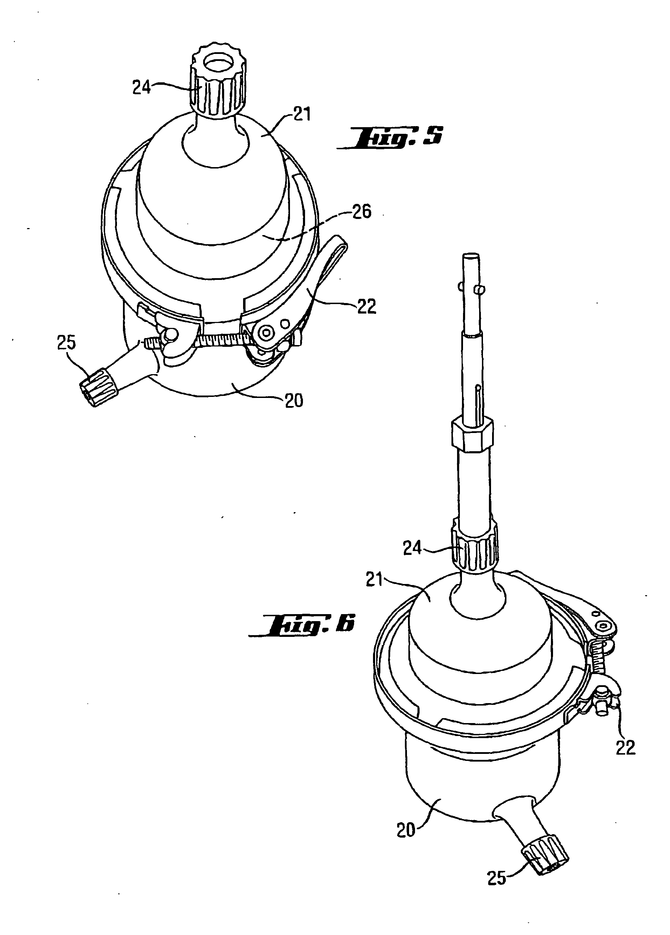

- Glass trap equipment *(Supplier: TECNOCHIMICA MODERNA s.r.l. Roma -ITALY),* constituted by the following parts (a disassembled view of the Glass trap can be seen in Figure 3, reference numerals relate thereto):
   1- Bottom part (20) (Nominal width_DN=60 mm, Volume= 500ml)
   2- Top part (21) (Nominal width_DN=60, Volume= 150ml)
   3- Aluminum Clip (22) for closure (Nominal width_DN=60)
   4- Silicon O-ring (23) (Nominal width_DN=60)
   5- Hole cup (24 in Figure 6) (Thread_SVL 15)
   6- Silicone Septa (25)
- Solid phase micro extraction (SPME) fiber coated with 100 µm polydimethylsiloxane (PDMS) *(Supplier: Sigma-Aldrich lSupelco Milano-ITALY, cod. number = 57300-U)*
- 100 µl micro syringe
- Gas chromatography instruments *(GC Trace-Thermofinnigan)* hyphenated with Mass Spectrometry (MS) *(PolarisQ)* detector or Flame ionization detector (FID)
- Capillary column J&W DB1: L=60 m, ID=0.32 mm, FT=0.25 µm. *(cod. number123-1062)*

### 2.Chemicals

- Menthyl lactate *(Supplier: Symrise former Haarmann & Reimer P.O.Box 1253 D-37601 Holzminden*/*Germany)*
- Polyethyleneglycol PEG400 *(supplier: CLARIANT GmbH 65840 Sulzbach am Taunus*/*Germany)*
- Mixture of 30 % by weight of menthyl lactate and 70% by weight of PEG-400

### 3.Experimental conditions

For assembling the glass trap equipment one septum (25) and the hole cup ((24) in Figure 6) are screwed into the top hole of the top part (21). Another septum is screwed into the hole of the bottom part (20). The O-ring (23) is placed on the top of the bottom part (20) as can be seen in Figure 4. The package material (26) to be tested is placed onto the O-ring (23), which must be completely covered by the package material (26). The top part (21) is placed on the bottom part such that the openings of the top (21) and the bottom part (20) are corresponding coextensively, whereby the package material (26) is enclosed between the top (21) and the bottom part (20) of the glass trap hermetically. The hermetical sealing of the top part (21) and the bottom part (20) is ensured by the clip (22), which is then applied to the assembled glass trap. The completely assembled glass trap with package material inside can be seen in Figure 5. After this 500 µl of the mixture of 70% by weight of PEG-400 and 30% by weight of menthyl lactate are injected into the bottom part (20) through the septum by a microliter syringe. Subsequently, the glase trap is placed into an oven and maintained at 40°C for 2 hours. After this the menthyl lactate present in the top part (21) of the glass trap is sampled by the SPME fibre for 20 min. The sampling step is illustrated in Figure 6. The SPME fibre is then desorbed for 1 min into the injector port of the gas chromatography apparatus. The gas chromatography run is acquired with either an FID or a MS detector.

### 4. Performance measure

The permeabilities are calculated versus a reference tested with the same amount of PEG-400/menthyl lactate mixture but without any package material between the top and the bottom part. This reference represents 100% permeability. The test runs with package material deployed between top and bottom part of the glass trap are between 0% and 100% accordingly, depending on their barrier performance.

### 5. Results

The above test has been done with Nordenia 6261BK packaging material and with conventional polyethylene packaging material Nordenia BK 5432 for comparison. Nordenia 6162BK resulted in 5% permeability towards menthyl lactate whereas the polyethylene material Nordenia BK 5432 resulted in 65% permeability towards menthyl lactate.

## Claims

1. A packaged absorbent article for feminine hygiene wherein sai absorbent article is selected from a sanitary napkin, a panty liner, an interlabial device, a tampon or an incontinence device, said absorbent article being packaged in a vapour-impermeable package made of a package material; wherein said package completely encloses said absorbent article and can prevent contact of said absorbent article with the outside environment; said packaged absorbent article being **characterized in that** said absorbent article comprises a nor -aqueous volatile material having a vapour pressure at 20°C of at least 0.01 mbar, and said package material has a thickness comprised between 0.020 and 1 millimeters and a basis weight comprised between 18.0 and 60.0 g/m² and further com rises at least one barrier layer made of a material selected from the group consisting of ethyl vinyl alcohol resins, aluminised film and aluminum foil, oriented polyethylene terephtalate, glycol-modified polyethylene terephtalate, oriented polyamide, aromatic polyamide, polymeric films being coated with volatile-impermeable lacquers or mixtures thereof; and wherein said package material has a permeability with respect to menthyl lactate of not more than 25 % according to the permeability test described herein.

2. The packaged article of claim 1, **characterized in that** said non-aqueous volatile material is a cooling agent able to stimulate thermo-receptors of the skin and/or mucosal surface of the wearer of said article, to convey a freshness sensation to the wearer, without the need to change the temperature on the skin and/or mucosal surface.

3. The packaged article of claim 2, **characterized in that** said cooling agent is selected from the chemical classes consisting of ketals, carboxamides, cyclohexyl derivatives, cyclohexanol derivatives, camphor, borneol, eucalyptol, methyl salicylate, tea tree oil, eucalyptus oil, peppermint oil, or mixtures thereof.

4. The packaged article of any of the preceding claims, **characterized in** said non-aqueous volatile material is selected from the group consisting of:
(i) a ketal according to the following formula: 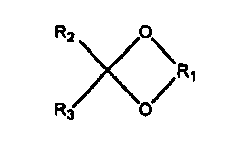 in which R¹ represents a C₂-C₆-alkylene radical having at least 1, but not more than 3 hydroxyl group(s), preferably 1 hydroxyl group and either R² and R² independently of one another represent C₁-C₁₀-alkyl which is optionally substituted by 1 to 3, radicals selected from the group comprising hydroxyl, amino and halogen (such as fluorine, chlorine, bromine or iodine), C₅-C₇-cycloalkyl, preferably cyclohexyl, C₆ -C₁₂-aryl, preferably phenyl, with the proviso that the total number of the C atoms of R² and R³ is not less than 3, or R² and R³ together represent an alkylene radical which, together with the carbon atom which carries the radicals R² and R³, forms a 5-7-membered ring, while it is possible for this alkylene radical, in turn, to be substituted by C₁-C₆-alkyl groups, or mixtures thereof; or
(ii) a carboxamide of the following formula: 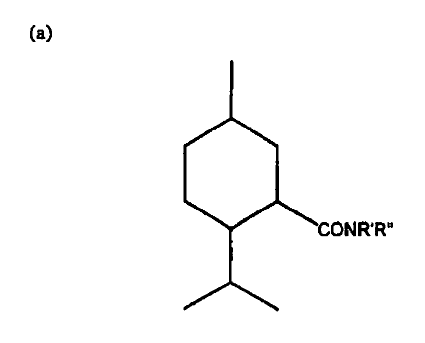 wherein R', when taken separately, is hydrogen or an aliphatic radical containing up to 25 carbon atoms; R" when taken separately is hydroxy, or an aliphatic radical containing up to 25 carbon atoms, with the proviso that when R' is hydrogen R" may also be an aryl radical of up to 10 carbon atoms and selected from the group consisting of substituted phenyl, phenalkyl or substituted phenalkyl, naphthyl and substituted naphthyl, pyridyl; and R' and R", when taken together with the nitrogen atom to which they are attached, represent a cyclic or heterocyclic group of up to 25 carbon atoms, or mixtures thereof; or 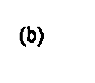 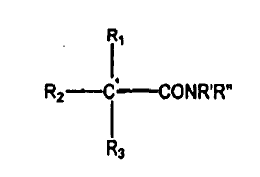 wherein R' and R", when taken separately, are each hydrogen, C₁-C₅ alkyl or C₁-C₈ hydroxyalkyl and provide a total of no more than 8 carbon atoms, with the proviso that when R' is hydrogen R" may also be alky carboxyalkyl of up to 6 carbon atoms; R' and R", when taken together, represent an alkylene group of up to 6 carbon atoms, the opposite ends of which group are attached to the amide nitrogen atom thereby to form a nitrogen heterocycle, the carbon chain of which may optionally be interrupted by oxygen; R₁ is hydrogen or C₁-C₅ alkyl; and R₂ and R₃ are each C₁-C₅ alkyl; with the provisos that (i) R₁, R₂ and R₃ together provide a total of at least 5 carbon atoms, preferably from 5-10 carbon atoms; and (ii) when R₁ is hydrogen, R₂ is C₂-C₅ alkyl and R₃ is C₃-C₅ alkyl and at least one of R₂ and R₃ is branched, preferably in alpha or beta position relative to the carbon atom marked (*) in the formula, or mixture thereof; or
(iii) a cyclohexanol derivative according to the following formula: 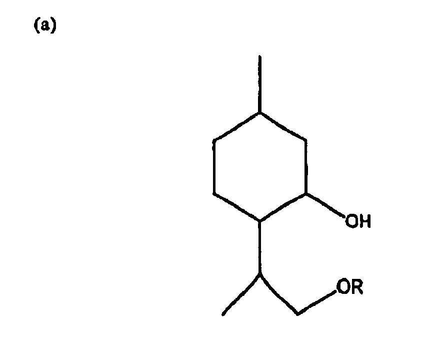 wherein R represents a linear or branched alkyl group having 1 to 5 carbon atoms; or 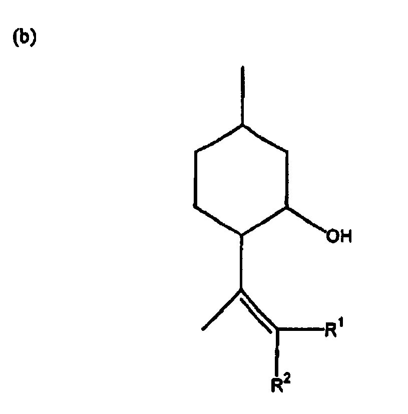 wherein R¹ and R² are independently hydrogen, or a linear or branched alkyl group having 1 to 5 carbon atoms, or mixtures thereof; or
(iv) or a cyclohexyl derivative according to the following formula: 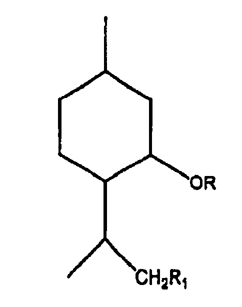 wherein R represents hydrogen, a C₁-C₅ linear or branched alkyl group, a C₁-C₅ alkenyl group, a C₁-C₅ alkoxy group or a C₁-C₅ acyloxy group, R₁ represents hydrogen, or a linear or branched alkyl group having from 1 to 5 carbon atoms; or
(v) mixtures thereof.

5. The packaged article of any of the preceding claims, **characterized in that** said non-aqueous volatile material is menthyl lactate.

6. The packaged article of claim 1, **characterized in that** said non-aqueous volatile material is a perfume or a fragrance for delivering a pleasant smell to the wearer of said article.

7. The packaged article of any of the preceding claims, **characterized in that** said non-aqueous volatile material is present in said article pure or in a delivery system, such as microcapsules or mixtures with polyethylene glycol.

8. The packaged article of any of the preceding claims, **characterized in that** said package material has a permeability of not more 15 % according to the permeability test described herein with respect to menthyl lactate.

9. The packaged article of any of the preceding claims, **characterized in that** said package material comprises at least one additional layer being made of a material selected from polyethylene (PE), polypropylene (PP), polyvinyl chloride (PVC) or combinations thereof.

10. The packaged article of any of the preceding claims, **characterized in that** said barrier layer is located between the inner and the outer surface layers of said package, said inner surface being the surface of said package, which is directly adjacent to said packaged article, said outer surface being the surface of said package, which is directly adjacent to the outside environment.

11. The packaged article of claim 10, **characterized in that** said barrier layer is positioned in a multilayer structure with 2 layers of polyethylene being arranged on each surface of said barrier layer.

12. The packaged article of claims 1-9, **characterized in that** said barrier layer forms said inner surface of said package.

13. The packaged article of any of the preceding claims, **characterized in that** said package material is a multi-layered co-extruded material.

14. The packaged article of any of the preceding claims, **characterized in that** said package is fully sealed, typically with impervious joins, seals, lines of adhesive or combinations thereof to prevent exposure of said article to the outside environment.

15. The packaged article of any of the preceding claims, **characterized in that** said article is individually packaged in said package.

16. The packaged article of claims 1-14, **characterized in that** said article is packaged in said package together with other articles of the same kind.

## Patentansprüche

1. Verpackter Absorptionsartikel für die weibliche Intimhygiene, wobei der Absorptionsartikel ausgewählt ist aus einer Damenbinde, einer Slipeinlage, einer Interlabialvorrichtung, einem Tampon oder einer Inkontinenzvorrichtung, wobei der Absorptionsartikel in einer dampfundurchlässigen Verpackung verpackt ist, die aus einem Verpackungsmaterial besteht; wobei die Verpackung den Absorptionsartikel vollständig einschließt und einen Kontakt des Absorptionsartikels mit der Außenumgebung verhindern kann; wobei der verpackte Absorptionsartikel **dadurch gekennzeichnet ist, dass** der Absorptionsartikel eine nicht-wässrige flüchtige Substanz mit einem Dampfdruck bei 20 °C von mindestens 1 Pa (0,01 mBar) umfasst und das Verpackungsmaterial eine Dicke von 0,020 bis 1 Millimeter und ein Grundgewicht von 18,0 bis 60,0 g/m² aufweist und ferner mindestens eine Sperrschicht umfasst, die aus einem Material besteht, das ausgewählt ist aus der Gruppe bestehend aus Ethylvinylalkoholharzen, aluminisierter Folie und Aluminiumfolie, gerecktem Polyethylenterephthalat, Glycol-modifiziertem Polyethylenterephthalat, gerecktem Polyamid, aromatischem Polyamid, Polymerfolien, die mit für flüchtige Substanzen undurchlässigen Lacken beschichtet sind, oder Mischungen davon; und wobei das Verpackungsmaterial eine Durchlässigkeit für Methyllactat von nicht mehr als 25 % gemäß den hierin beschriebenen Durchlässigkeitstests aufweist.

2. Verpackter Artikel nach Anspruch 1, **dadurch gekennzeichnet, dass** die nicht-wässrige flüchtige Substanz ein Kühlmittel ist, das in der Lage ist, Thermorezeptoren der Haut- und/oder der Schleimhautoberfläche der Trägerin des Artikels zu stimulieren, um der Trägerin ein Gefühl der Frische zu vermitteln, ohne dass die Temperatur auf der Haut- und/oder Schleimhautoberfläche geändert werden müsste.

3. Verpackter Artikel nach Anspruch 2, **dadurch gekennzeichnet, dass** das Kühlmittel ausgewählt ist aus den chemischen Klassen, die aus Ketalen, Carboxamiden, Cyclohexylderivaten, Cyclohexanolderivaten, Kampfer, Borneol, Eucalyptol, Methylsalicylat, Teebaumöl, Eukalyptusöl, Pfefferminzöl oder Mischungen davon bestehen.

4. Verpackter Artikel nach einem der vorstehenden Ansprüche, **dadurch gekennzeichnet, dass** das nicht-wässrige flüchtige Material ausgewählt ist aus der Gruppe bestehend aus:
(i) einem Ketal gemäß der folgenden Formel: 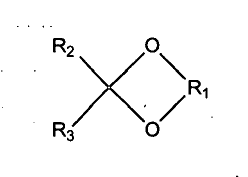 worin R¹ einen C₂-C₆-Alkylenrest mit mindestens 1, aber nicht mehr als 3 Hydroxylgruppen, vorzugsweise mit 1 Hydroxylgruppe darstellt, und sowohl R² als auch R³ unabhängig voneinander C₁-C₁₀-Alkyl darstellen, das optional mit 1 bis 3 Resten substituiert ist, die ausgewählt sind aus der Gruppe bestehend aus Hydroxyl, Amino und Halogen (wie Fluor, Chlor, Brom oder Iod), C₅-C₇-Cycloalkyl, vorzugsweise Cyclohexyl, C₆ -C₁₂-Aryl, vorzugsweise Phenyl, mit der Maßgabe, dass die Gesamtzahl der C-Atome von R² und R³ nicht kleiner als 3 ist, oder dass R² und R³ gemeinsam einen Alkylenrest darstellen, der zusammen mit dem Kohlenstoffatom, welches die Reste R² und R³ trägt, einen 5- bis 7-gliedrigen Ring bildet, während dieser Alkylenrest wiederum mit C₁-C₆-Alkylgruppen oder Mischungen davon substituiert sein kann; oder
(ii) ein Carboxamid der folgenden Formel: 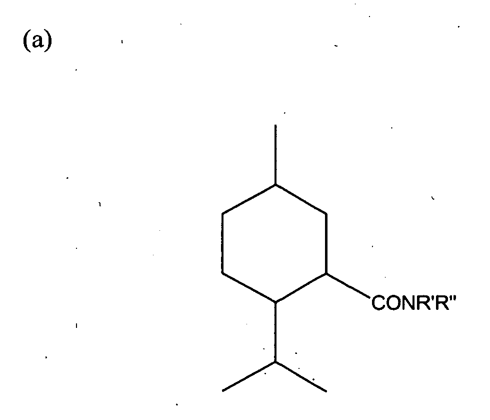 worin R', für sich betrachtet, Wasserstoff oder ein aliphatischer Rest mit bis zu 25 Kohlenstoffatomen ist; R", für sich betrachtet, Hydroxy oder ein aliphatischer Rest ist, der bis zu 25 Kohlenstoffatome enthält, mit der Maßgabe, dass R", wenn R' Wasserstoff ist, auch ein Arylrest mit bis zu 10 Kohlenstoffatomen sein kann und ausgewählt sein kann aus der Gruppe bestehend aus substituiertem Phenyl, Phenalkyl oder substituiertem Phenalkyl, Naphthyl und substituiertem Naphthyl, Pyridyl; und R' und R" gemeinsam mit dem Stickstoffatom, an das sie gebunden sind, eine zyklische oder heterozyklische Gruppe mit bis zu 25 Kohlenstoffatomen oder Mischungen davon darstellen; oder 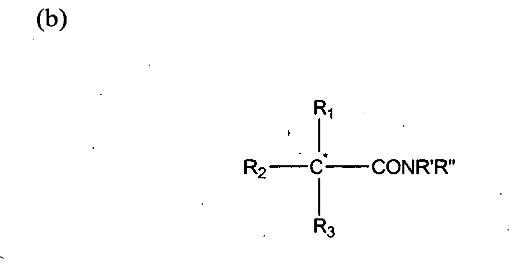 wobei R' und R", für sich betrachtet, jeweils C₁-C₅-Alkyl oder C₁-C₈-Hydroxyalkyl sind und insgesamt nicht mehr als 8 Kohlenstoffatome liefern, mit der Maßgabe, dass R", wenn R' Wasserstoff ist, auch Alkylcarboxyalkyl mit bis zu 6 Kohlenstoffatomen sein kann; R' und R", wenn sie zusammen genommen werden, für eine Alkylengruppe mit bis zu 6 Kohlenstoffatomen stehen, die gegenüber liegenden Enden der Gruppe an das Stickstoffatom des Amids gebunden sind, um **dadurch** einen Stickstoffheterocyclus zu bilden, die Kohlenstöfflcette davon wahlweise durch Sauerstoff unterbrochen sein kann; R₁ Wasserstoff oder C₁-C₅-Alkyl ist; und R₂ und R₃ jeweils C₁-C₅-Alkyl sind; mit der Maßgabe, dass (i) R₁, R₂ und R₃ zusammen eine Gesamtanzahl von mindestens 5 Kohleristoffatomen, vorzugsweise von 5 bis 10 Kohlenstoffatomen liefern; und (ii), wenn R₁ Wasserstoff ist, R₂ dann C₂-C₅-Alkyl ist und R₃ C₃-C₅-Alkyl ist, und mindestens einer von R₂ und R₃ verzweigt ist, vorzugsweise in einer alpha- oder beta-Position in Bezug auf das in der Formel mit (*) markierte Kohlenstoffatom, oder eine Mischung davon; oder
(iii) ein Cyclohexanolderivat gemäß der folgenden Formel: 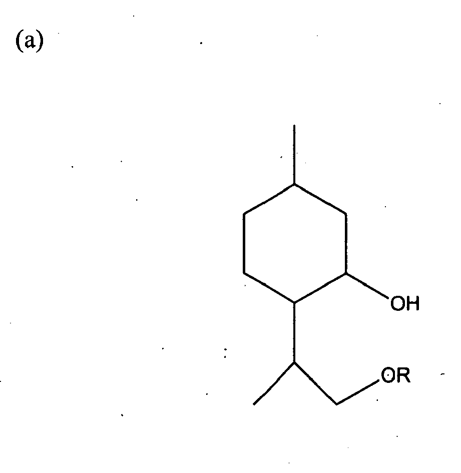 wobei R eine lineare oder verzweigte Alkylgruppe mit 1 bis 5 Kohlenstoffatomen darstellt; oder 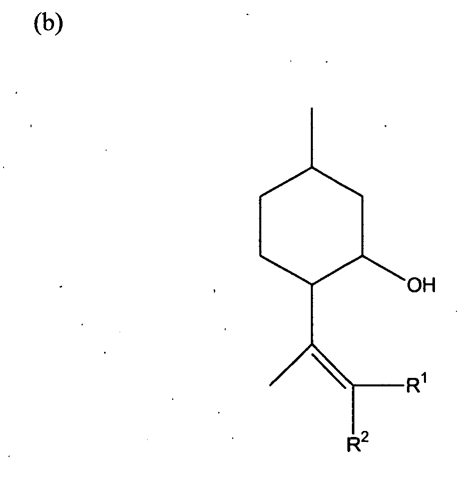 worin R¹ und R² unabhängig voneinander Wasserstoff oder eine lineare oder verzweigte Alkylgruppe mit 1 bis 5 Kohlenstoffatomen oder Mischungen davon sind; oder
(iv) oder ein Cyclohexylderivat gemäß der folgenden Formel: 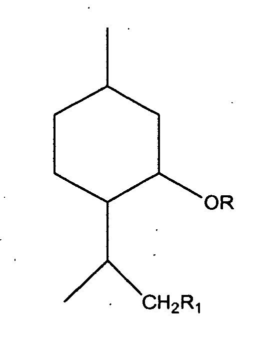 worin R Wasserstoff, eine lineare oder verzweigte C₁-C₅-Alkylgruppe, eine C₁-C₅- Alkenylgruppe, eine C₁-C₅- Alkoxygruppe oder eine C₁-C₅- Acyloxygruppe darstellt, R₁ Wasserstoff oder eine lineare oder verzweigte Alkylgruppe mit 1 bis 5 Kohlenstoffatomen darstellt; oder (v) Mischungen davon.

5. Verpackter Artikel nach einem der vorstehenden Ansprüche, **dadurch gekennzeichnet, dass** die nicht-wässrige flüchtige Substanz Menthyllactat ist.

6. Verpackter Artikel nach Anspruch 1, **dadurch gekennzeichnet, dass** die nicht-wässrige flüchtige Substanz ein Parfüm oder ein Duftstoff ist, das bzw. der einen angenehmen Duft für die Trägerin des Artikels abgeben soll.

7. Verpackter Artikel nach einem der vorstehenden Ansprüche, **dadurch gekennzeichnet, dass** die nicht-wässrige flüchtige Substanz in dem Artikel in reiner Form oder in einem Abgabesystem, wie in Mikrokapseln oder in Mischungen mit Polyethylenglycol, vorliegt.

8. Verpackter Artikel nach einem der vorstehenden Ansprüche, **dadurch gekennzeichnet, dass** das Verpackungsmaterial eine Durchlässigkeit von 15 % gemäß den hierin beschriebenen Durchlässigkeitstests in Bezug auf Menthyllactat hat.

9. Verpackter Artikel nach einem der vorstehenden Ansprüche, **dadurch gekennzeichnet, dass** das Verpackungsmaterial mindestens eine zusätzliche Schicht umfasst, die aus einem Material besteht, das ausgewählt ist aus Polyethylen (PE), Polypropylen (PP), Polyvinylchlorid (PVC) oder Kombinationen davon.

10. Verpackter Artikel nach einem der vorstehenden Ansprüche, **dadurch gekennzeichnet, dass** die Sperrschicht sich zwischen den inneren und äußeren Oberflächenschichten der Verpackung befindet, wobei die innere Oberfläche diejenige Oberfläche der Verpackung ist, die direkt an den verpackten Artikel angrenzt, wobei die Außenfläche diejenige Oberfläche der Verpackung ist, die direkt an die Außenumgebung angrenzt.

11. Verpackter Artikel nach Anspruch 10, **dadurch gekennzeichnet, dass** die Sperrschicht in einer Mehrschichtstruktur angeordnet ist, in der 2 Schichten aus Polyethylen auf jeder Oberfläche der Sperrschicht angeordnet sind.

12. Verpackter Artikel nach einem der Ansprüche 1 bis 9, **dadurch gekennzeichnet, dass** die Sperrschicht die innere Oberfläche der Verpackung bildet.

13. Verpackter Artikel nach einem der vorstehenden Ansprüche, **dadurch gekennzeichnet, dass** das Verpackungsmaterial ein mehrschichtiges co-extrudiertes Material ist.

14. Verpackter Artikel nach einem der vorstehenden Ansprüche, **dadurch gekennzeichnet, dass** die Verpackung vollständig abgedichtet ist, in der Regel mit undurchlässigen Verbindungen, Dichtungen, Klebstofflinien oder Kombinationen davon, um zu verhindern, dass der Artikel der Außenumgebung ausgesetzt wird.

15. Verpackter Artikel nach einem der vorstehenden Ansprüche, **dadurch gekennzeichnet, dass** der Artikel in der Verpackung einzeln verpackt ist.

16. Verpackter Artikel nach einem der Ansprüche 1 bis 14, **dadurch gekennzeichnet, dass** der Artikel gemeinsam mit anderen Artikeln derselben Art in der Packung verpackt ist.

## Revendications

1. Article absorbant pour hygiène féminine conditionné, où ledit article absorbant est choisi parmi une serviette hygiénique, un protège-slip, un dispositif interlabial, un tampon ou un dispositif pour l'incontinence, ledit article absorbant étant conditionné dans un conditionnement imperméable à la vapeur constitué d'un matériau de conditionnement ; dans lequel ledit conditionnement enferme complètement ledit article absorbant et peut empêcher un contact dudit article absorbant avec l'environnement extérieur ; ledit article absorbant conditionné étant **caractérisé en ce que** ledit article absorbant comprend un matériau volatil non aqueux ayant une pression de vapeur à 20 °C d'au moins 1 Pa (0,01 mbar), et ledit matériau de conditionnement a une épaisseur comprise entre 0,020 et 1 millimètre et une masse surfacique comprise entre 18,0 et 60,0 g/m² et comprend en outre au moins une couche de barrière constituée d'un matériau choisi dans le groupe constitué de résines d'alcool éthyl vinylique, de film aluminisé et papier aluminium, de polyéthylène téréphtalate orienté, de polyéthylène téréphtalate modifié au glycol, de polyamide orienté, de polyamide aromatique, de films polymères qui sont revêtus de laques imperméables volatiles ou de leurs mélanges ; et dans lequel ledit matériau de conditionnement a une perméabilité par rapport au lactate de menthyle de pas plus de 25 % selon le test de perméabilité décrit ici.

2. Article conditionné selon la revendication 1, **caractérisé en ce que** ledit matériau volatil non aqueux est un agent de refroidissement capable de stimuler les thermorécepteurs de la peau et/ou de la surface muqueuse du porteur dudit article, pour véhiculer une sensation de fraîcheur au porteur, sans le besoin de changer la température sur la peau et/ou la surface muqueuse.

3. Article conditionné selon la revendication 2, **caractérisé en ce que** ledit agent de refroidissement est choisi parmi les classes chimiques constituées de cétals, carboxamides, dérivés de cyclohexyle, dérivés de cyclohexanol, camphre, boméol, eucalyptol, salicylate de méthyle, huile de théier, huile d'eucalyptus, essence de menthe poivrée, ou leurs mélanges.

4. Article conditionné selon l'une quelconque des revendications précédentes, **caractérisé en ce que** ledit matériau volatil non aqueux est choisi dans le groupe constitué de :
(i) un cétal selon la formule suivante : 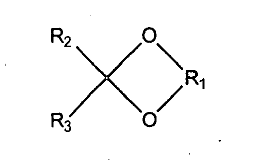 dans laquelle R¹ représente un radical alkylène en C₂ à C₆ ayant au moins 1, mais pas plus de 3 groupe(s) hydroxyle, de préférence 1 groupe hydroxyle, et l'un ou l'autre de R² et R³ indépendamment l'un de l'autre représente un alkyle en C₁ à C₁₀ qui est éventuellement substitué par 1 à 3 radicaux choisis parmi le groupe comprenant hydroxyle, amino et halogène (tel que fluor, chlore, brome ou iode), un cycloalkyle en C₅ à C₇, de préférence un cyclohexyle, un aryle en C₆ à C₁₂, de préférence un phényle, à condition que le nombre total des atomes de C de R² et R³ ne soit pas moins de 3, ou R² et R³ représentent conjointement un radical alkylène qui, conjointement avec l'atome de carbone qui porte les radicaux R² et R³, forme un cycle de 5 à 7 éléments, étant possible que ce radical alkylène, à son tour, soit substitué par des groupes alkyle en C₁ à C₆, ou leurs mélanges ; ou
(ii) un carboxamide de formule suivante : 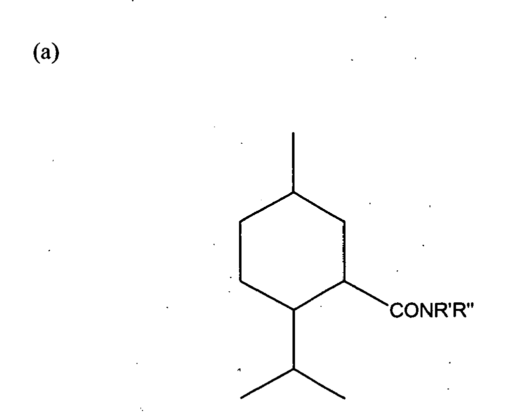 dans laquelle R', lorsqu'il est pris séparément, est un hydrogène ou un radical aliphatique contenant jusqu'à 25 atomes de carbone ; R" lorsqu'il est pris séparément est un hydroxy, ou un radical aliphatique contenant jusqu'à 25 atomes de carbone, à condition que lorsque R' est un hydrogène, R" peut également être un radical aryle allant jusqu'à 10 atomes de carbone et choisi dans le groupe constitué de phényle substitué, phénalkyle ou phénalkyle substitué, naphtyle et naphtyle substitué, pyridyle ; et R' et R", lorsqu'ils sont pris ensemble avec l'atome d'azote auquel ils sont fixés, représentent un groupe cyclique ou hétérocyclique allant jusqu'à 25 atomes de carbone, ou leurs mélanges ; ou 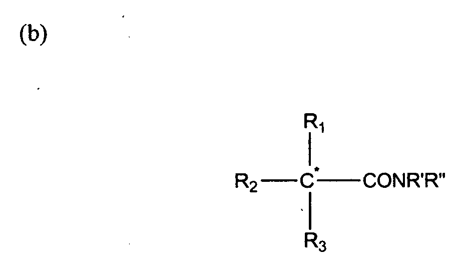 où R' et R", lorsqu'ils sont pris séparément, sont chacun un hydrogène, un alkyle en C₁ à C₅ ou un hydroxyalkyle en C₁ à C₈ et fournissent un total d'au plus 8 atomes de carbone, à condition que, lorsque R' est un hydrogène, R" peut également être un alkylcarboxyalkyle de jusqu'à 6 atomes de carbone ; R' et R", pris conjointement, représentent un groupe alkylène de jusqu'à 6 atomes de carbone, groupe dont les extrémités opposées sont fixées à l'atome d'azote d'amide de façon à former de ce fait un hétérocycle azoté dont la chaîne carbonée peut éventuellement être interrompue par un oxygène ; R₁ est un hydrogène ou un alkyle en C₁ à C₅ ; et R₂ et R₃ sont chacun un alkyle en C₁ à C₅ ; à condition que (i) R₁, R₂ et R₃ fournissent conjointement un total d'au moins 5 atomes de carbone, de préférence de 5 à 10 atomes de carbone ; et (ii) lorsque R₁ est un hydrogène, R₂ est un alkyle en C₂ à C₅ et R₃ est un alkyle en C₃ à C₅ et au moins l'un parmi R₂ et R₃ est ramifié, de préférence dans une position alpha ou bêta par rapport à l'atome de carbone marqué (*) dans la formule, ou un mélange de ceux-ci ; ou
(iii) un dérivé de cyclohexanol selon la formule suivante : 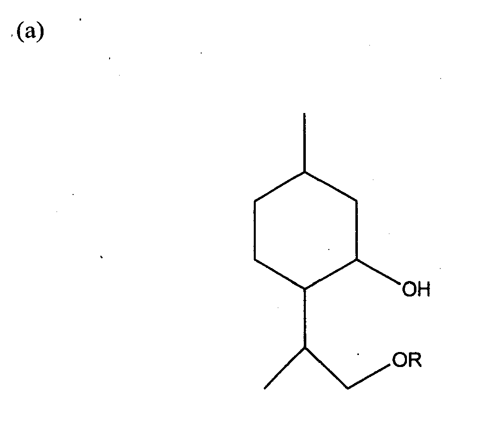 où R représente un groupe alkyle linéaire ou ramifié ayant 1 à 5 atomes de carbone; ou 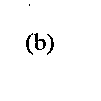 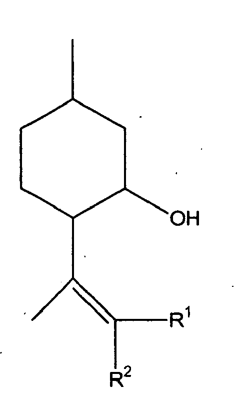 où R¹ et R² sont indépendamment un hydrogène, ou un groupe alkyle linéaire ou ramifié ayant 1 à 5 atomes de carbone, ou leurs mélanges ; ou
(iv) ou un dérivé de cyclohexyle selon la formule suivante : 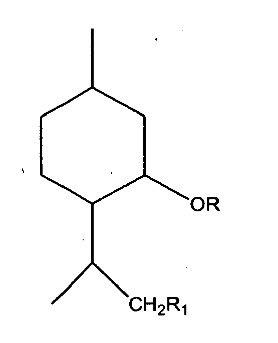 dans laquelle R représente un hydrogène, un groupe alkyle linéaire ou ramifié en C₁ à C₅, un groupe alcényle en C₁ à C₅, un groupe alcoxy en C₁ à C₅ ou un groupe acyloxy en C₁ à C₅ , R₁ représente un hydrogène, ou un groupe alkyle linéaire ou ramifié ayant de 1 à 5 atomes de carbone ; ou
(v) leurs mélanges.

5. Article conditionné selon l'une quelconque des revendications précédentes, **caractérisé en ce que** ledit matériau volatil non aqueux est du lactate de menthyle.

6. Article conditionné selon la revendication 1, **caractérisé en ce que** ledit matériau volatil non aqueux est un parfum ou une fragrance pour libérer une odeur plaisante au porteur dudit article.

7. Article conditionné selon l'une quelconque des revendications précédentes, **caractérisé en ce que** ledit matériau volatil non aqueux est présent dans ledit article pur ou dans un système d'administration, tel que des microgélules ou des mélanges avec du polyéthylène glycol.

8. Article conditionné selon l'une quelconque des revendications précédentes, **caractérisé en ce que** ledit matériau de conditionnement a une perméabilité de pas plus de 15 % selon le test de perméabilité décrit ici par rapport au lactate de menthyle.

9. Article conditionné selon l'une quelconque des revendications précédentes, **caractérisé en ce que** ledit matériau de conditionnement comprend au moins une couche supplémentaire qui est constituée d'un matériau choisi parmi le polyéthylène (PE), le polypropylène (PP), le chlorure de polyvinyle (PVC) ou leurs combinaisons.

10. Article conditionné selon l'une quelconque des revendications précédentes, **caractérisé en ce que** ladite couche de barrière est située entre les couches de surfaces interne et externe dudit conditionnement, ladite surface interne étant la surface dudit conditionnement, qui est directement adjacente audit article conditionné, ladite surface externe étant la surface dudit conditionnement, qui est directement adjacente à l'environnement extérieur.

11. Article conditionné selon la revendication 10, **caractérisé en ce que** ladite couche de barrière est positionnée dans une structure multicouche avec 2 couches de polyéthylène qui sont arrangées sur chaque surface de ladite couche de barrière.

12. Article conditionné selon les revendications 1 à 9, **caractérisé en ce que** ladite couche de barrière forme ladite surface interne dudit conditionnement.

13. Article conditionné selon l'une quelconque des revendications précédentes, **caractérisé en ce que** ledit matériau de conditionnement est un matériau coextrudé multicouche.

14. Article conditionné selon l'une quelconque des revendications précédentes, **caractérisé en ce que** ledit conditionnement est complètement étanche, typiquement avec des jointures imperméables, des joints, des lignes d'adhésif ou leurs combinaisons pour empêcher une exposition dudit article à l'environnement extérieur.

15. Article conditionné selon l'une quelconque des revendications précédentes, **caractérisé en ce que** ledit article est conditionné individuellement dans ledit conditionnement.

16. Article conditionné selon les revendications 1 à 14, **caractérisé en ce que** ledit article est conditionné dans ledit conditionnement conjointement avec d'autres articles du même type.
